# EUROPÄISCHE PATENTSCHRIFT

(11) **EP 0 378 852 B1**
(45) Veröffentlichungstag und Bekanntmachung des Hinweises auf die Patenterteilung: **27.10.1993**
(21) Anmeldenummer: 89123919.6
(22) Anmeldetag: 27.12.1989
(51) Int. Cl.: A61K 37/02

(54) **Topisch anwendbare pharmazeutische Zubereitung**
Pharmaceutical preparation for topical use
Préparation pharmaceutique pour utilisation topique

(30) Priorität: 05.01.1989 DE 3900198
(43) Veröffentlichungstag der Anmeldung: 25.07.1990
(73) Patentinhaber: Drenk, Franz, Dr., D-30627 Hannover (DE)
(72) Erfinder: Drenk, Franz, Dr., D-30627 Hannover (DE)

(56) Entgegenhaltungen:
- EP-A- 0 131 150
- EP-A- 0 243 179
- US-A- 4 141 973
- EXPERIMENTAL CELL RESEARCH, Band 172, 1987, Seiten 92-100 New York, US. T. IMAMURA et al.: "Heparan sulfate and heparin as a potentiator or a suppressor of growth of normal and transformed vascular endothelial cells"

## Beschreibung

Die Erfindung betrifft eine neue topisch anwendbare pharmazeutische Zubereitung, enthaltend Fibroblast Growth Factor (FGF) und Hyaluronsäure und/oder eines ihrer physiologisch unbedenklichen Salze.

Mit "FGF" bezeichnet man eine Gruppe von basischem ("bFGF") oder sauren ("sFGF") Polypeptiden, die als Mitogene für Endothelzellen wirken und z.B. aus der Hypophyse und dem Gehirn insbesondere von Rindern isoliert werden können. Eine Verwendung von FGF zur Wundheilung in gewerblichem Maßstab ist bisher nicht bekannt.

Hyaluronsäure und ihre physiologisch unbedenklichen Salze, insbesondere das Natriumsalz, sind bisher insbesondere zur Volumensubstitution und zum Schutz des Gewebes bei chirurgischen Eingriffen am Auge verwendet worden, aber auch als Monosubstanz zur Wundheilung [vgl. z.B. S. ALEXANDER und R. DONOFF, J. Surg. Res. 29, 422-429 (1980), C. DOILLON und F. SILVER, Biomat. 7, 3-8 (1986); D. WEST et al., Science 228, 1324-1326 (1985); G. ABATANGELO et al., J. Surg. Res. 35, 410-416 (1983)] und für viele andere therapeutische Zwecke.

Die Therapie von Wunden, insbesondere die Therapie schlecht heilender Wunden, stellt auch heute noch ein großes Problem dar. Meist nimmt man nur eine symptomatische Behandlung vor.

Der Erfindung lag die Aufgabe zugrunde, neue topisch anwendbare pharmazeutische Zubereitungen mit verbesserter Wirksamkeit insbesondere bei der Wundheilung aufzufinden, die die Nachteile der bekannten Zubereitungen nicht oder nur in geringerem Maße zeigen.

Es wurde gefunden, daß Zubereitungen der angegebenen Art, die gleichzeitig FGF und Hyaluronsäure und/oder eines ihrer physiologisch unbedenklichen Salze enthalten, eine besonders gute und schnelle Wundheilung bewirken, insbesondere auch bei schlecht heilenden Wunden.

Gegenstand der Erfindung ist eine topisch anwendbare pharmazeutische Zubereitung, enthaltend FGF und Hyaluronsäure und/oder eines ihrer physiologisch unbenklichen Salze.

Ferner ist Gegenstand der Erfindung ein Verfahren zur Herstellung einer pharmazeutischen Zubereitung, dadurch gekennzeichnet, daß man FGF und Hyaluronsäure und/oder eines ihrer physiologisch unbedenklichen Salze zusammen mit mindestens einem festen, flüssigen oder halbflüssigen Träger- oder Hilfsstoff in eine für die topische Anwendung geeignete Dosierungsform bringt. Eine solche pharmazeutische Zubereitung kann bei der Wundheilung verwendet werden.

Mit den erfindungsgemäßen Zubereitungen lassen sich insbesondere Wunden behandeln, bei denen die normale Heilung durch eine gestörte Kapillarfunktion beeinträchtigt ist. Hierher gehören z.B. diabetische Mikroangiopathie, Raucherbein, Brandverletzungen, Sklerodermie, postthrombotische Ulcera, chronische Hautulcera, Decubitalulcera.

Als pharmazeutische Zubereitungen eignen sich insbesondere Gele, Salben, Cremes, Pasten, kolloidale Lösungen, Lotionen, Sprays, Schäume, Pflaster.

Sie können neben den Wirkstoffen übliche Träger- und/oder Hilfsstoffe enthalten, z.B. Wasser, Alkohole wie Ethanol, Isopropanol, Cetylalkohol, Carbonsäuren wie Palmitin- oder Stearinsäure, pflanzliche Öle, Paraffine, Benzylalkohole, Polyethylenglykole, Glycerinester wie Glycerintriacetat oder Glycerinmonostearat, Glykole wie 1,2-Propandiol oder Glycerin, Lanolin, Vaseline, weitere Matrixsubstanzen wie Kollagen, ferner Konservierungs-, Stabilisierungs- und/oder oberflächenaktive Substanzen wie z.B. Sorbitanfettsäureester oder Fettalkoholether des Polyoxyethylens.

Ein Zusatz weiterer Wirkstoffe ist ebenfalls möglich. Als solche eignen sich z.B. Antibiotika, Chemotherapeutika, Antimykotika.

Der Gehalt an FGF liegt in der Regel zwischen 10⁻⁵ und 1, vorzugsweise zwischen 10⁻⁴ und 10⁻³ Gewichtsprozent, derjenige an Hyaluronsäure bzw. deren Salzen zwischen 0,05 und 5, vorzugsweise zwischen 0,2 und 2 Gewichtsprozent. Unter den Salzen der Hyaluronsäure ist das Natriumsalz bevorzugt.

Besonders bevorzugt sind Ausführungsformen, die außer den beiden Wirkstoffen im wesentlichen nur Wasser enthalten und dementsprechend in Gelform vorliegen. In diesen Gelen kann die Hyaluronsäure als Trägersubstanz für FGF betrachtet werden; die Abwesenheit anderer Stoffe vermindert die Gefahr von Unverträglichkeitsreaktionen im Wundbereich, die sonst einen limitierenden Faktor bei der Therapie bilden.

### Beispiel 1: Gel

Man mischt unter sterilen Bedingungen 100 mg bFGF (aus Rinderhirn), 2 kg Hyaluronsäure-Natriumsalz und 99 kg Wasser und verwendet das erhaltene Gel unmittelbar zur Wundbehandlung.

### Beispiel 2: Gel

Man arbeit wie in Beispiel 1 angegeben, verwendet aber gentechnologisch hergestelltes sFGF.

### Beispiel 3: Creme

Die in üblicher Weise hergestellte Creme setzt sich wie folgt zusammen:

| | |
|---|---|
| sFGF | 100 mg |
| Hyaluronsäure | 1,0 kg |
| Paraffin, dickflüssig | 8,0 kg |
| Stearinsäure | 2,0 kg |
| Cetylalkohol | 4,0 kg |
| Glycerinmonostearat | 3,6 kg |
| Macrogolstearat | 2,4 kg |
| Parfüm | q.s. |
| Konservierungsmittel | q.s. |
| gereinigtes Wasser | ad 100,0 kg. |

### Beispiel 4: Creme

Man arbeitet wie in Beispiel 3 angegeben, verwendet aber 0,5 g bFGF und 2,0 kg Hyaluronsäure-Natriumsalz.

### Beispiel 5: Lotion

Die in üblicher Weise hergestellte Lotion setzt sich wie folgt zusammen:

| | |
|---|---|
| bFGF | 100 mg |
| Hyaluronsäure-Natriumsalz | 0,5 kg |
| Paraffin, dünnflüssig | 5,0 kg |
| Polyethylenglykol-5-stearylstearat | 5,0 kg |
| Polyoxyethylenstearylether (21 Mol EO) | 5,0 kg |
| Mittelkettige Triglyceride | 5,0 kg |
| Glycerin | 2,0 kg |
| Parfüm | q.s. |
| Konservierungsmittel | q.s. |
| gereinigtes Wasser | ad 100,0 kg. |

### Beispiel 6: Lotion

Man arbeitet wie in Beispiel 5 angegeben, verwendet aber nur 50 mg SFGF und 200 g Hyaluronsäure-Natriumsalz.

### Beispiel 7: Spray

Man bereitet ein Gemisch aus 0,5 kg Hyaluronsäure-Natriumsalz, 100 mg sFGF, 1 kg Polyvinylpyrrolidon und Wasser (ad 100 kg). 9 Gewichtsteile dieses Gemischs werden mit 1 Gewichtsteil Treibmittelgemisch zu dem gewünschten Spray verarbeitet. Das Treibmittelgemisch besteht aus Propan, Butan und Isobutan im Gewichtsverhältnis 1:1:1.

## Patentansprüche

1. Topisch anwendbare pharmazeutische Zubereitung, enthaltend Fibroblast Growth Factor (FGF) und Hyaluronsäure und/oder eines ihrer physiologisch unbenklichen Salze.

2. Verfahren zur Herstellung einer pharmazeutischen Zubereitung gemäß Anspruch 1, dadurch gekennzeichnet, daß man FGF und Hyaluronsäure und/oder eines ihrer physiologisch unbedenklichen Salze zusammen mit mindestens einem festen, flüssigen oder halbflüssigen Träger- oder Hilfsstoff in eine für die topische Anwendung geeignete Dosierungsform bringt.

## Claims

1. Pharmaceutical preparations , suitable for topical application, containing fibroblast growth factor (FGF) and hyaluronic acid and/or one of its physiologically acceptable salts.

2. Method for the production of a pharmaceutical preparation according to Claim 1, characterized in that FGF and hyaluronic acid and/or one of its physiologically acceptable salts are brought into a suitable dosage form for topical application together with at least one solid, liquid or semi - liquid excipient or auxiliary.

## Revendications

1. La préparation pharmaceutique à utilisation topique contenant le Fibroblast Growth Factor (FGF) et l'acide hyaluronique et/ ou l'un de ses sels physiologiques non toxiques.

2. Le procedé de fabrication d'une préparation pharmaceutique selon les conditions énumérées en 1, caracterisé par le fait que le FGF et l'acide hyaluronique et/ ou l'un de ses sels physiologiques non toxiques, associés à au moins un porteur ou substance auxilliaire, solide, liquide, ou semi-liquide, ait une concentration telle, qu'ils puissent être à utilisation topique.
